# EUROPÄISCHE PATENTANMELDUNG

(11) **EP 4 643 649 A1**
(43) Veröffentlichungstag der Anmeldung: **05.11.2025**
(21) Anmeldenummer: 25173933.0
(22) Anmeldetag: 02.05.2025
(51) Int. Cl.: A23K 10/16, A23K 20/158, A23K 20/174, A23K 50/40, A61P 1/16, A61P 1/18, A61P 3/04, A61P 13/12, A61P 17/00, A61P 37/08

(54) **VEGANE TIERFUTTERMITTELZUSAMMENSETZUNG**

(30) Priorität: 03.05.2024 DE 102024112469
(71) Anmelder: younikat GmbH, 82396 Pähl (DE)
(72) Erfinder: KNECHTEL, Julia, 90762 Fürth (DE); STEFFEN, Carla, 82234 Wessling (DE); ZAUNE-FIGLAR, Tessa, 82396 Pähl (DE)
(74) Vertreter: Kehl, Ascherl, Liebhoff & Ettmayr Patentanwälte Partnerschaft mbB

(57) **Zusammenfassung**

Die Erfindung betrifft eine vegane Tierfuttermittelzusammensetzung, die ein Einzelzellproteinprodukt aus der Biomasse einer Mikroorganismusspezies enthält, zur Verwendung in einem Verfahren zur Behandlung von Futtermittelallergien und entzündlichen Darmerkrankungen sowie von Komorbiditäten bei Tieren.

## Beschreibung

Die Erfindung betrifft eine vegane Tierfuttermittelzusammensetzung, die ein Einzelzellproteinprodukt aus der Biomasse einer Mikroorganismusspezies enthält, zur Verwendung in einem Verfahren zur Behandlung von Futtermittelallergien und entzündlichen Darmerkrankungen sowie Komorbiditäten bei Tieren.

Futtermittelallergien bei Tieren sind eine Überempfindlichkeitsreaktion des Immunsystems auf bestimmte Bestandteile in der Nahrung. Diese Reaktion kann von milden Symptomen wie Juckreiz bis hin zu schwerwiegenden Reaktionen wie Erbrechen, Durchfall, und Hautausschlägen reichen.

### Pathogenese von Futtermittelallergien

Eine Futtermittelallergie bei Hunden und Katzen entsteht, wenn das Immunsystem bestimmte Nahrungsbestandteile fälschlicherweise als schädlich einstuft und daraufhin eine Abwehrreaktion einleitet. Dieser Prozess ähnelt dem bei Menschen und verläuft in zwei Hauptphasen:
- **Sensibilisierungsphase:** Bei der ersten Aufnahme des Allergens erkennt das Immunsystem dieses als fremd und produziert spezifische Antikörper, insbesondere Immunglobulin E (IgE). Diese Antikörper binden an Mastzellen und Basophile, die bei allergischen Reaktionen eine zentrale Rolle spielen.
- **Auslösephase:** Bei erneutem Kontakt mit dem Allergen bindet dieses an die bereits gebundenen IgE-Antikörper auf den Mastzellen und Basophilen. Dies führt zur Freisetzung von Entzündungsmediatoren wie Histamin, die für die typischen allergischen Symptome verantwortlich sind.
- Eine Futtermittelallergie kann demnach nur gegen Allergene bestehen, die dem Körper bereits bekannt sind. Innovative Proteinquellen, gegen die noch keine Exposition bestand, sind demnach optimal für Hunde mit Futtermittelallergien geeignet.

### Literatur:

- Sampson, H. A. (1999). Food allergy. Part 1: immunopathogenesis and clinical disorders. Journal of Allergy and Clinical Immunology, 103(5), 717-728.
- Verlinden, A., Hesta, M., Millet, S., & Janssens, G. P. J. (2006). Food allergy in dogs and cats: a review. Critical reviews in food science and nutrition, 46(3), 259-273.

### Nieren, Leber- und Pankreaserkrankungen und Diätetik zur Behandlung Nierenerkrankungen:

- Reduktion der Phosphorzufuhr (abhängig vom Grad der Nierenerkrankung) und Verwendung ausschließlich organischer Phosphatquellen (versus anorganische Phosphatquellen) Lebererkrankungen:
- moderate Vitamin-A- und Kupferzufuhr

### Niere und Leber:

- Reduktion der Proteinzufuhr
- Verwendung von hochverdaulichen Proteinquellen zur Reduktion von Stoffwechselprodukten, die die Nieren/Leber belasten können
- Verwendung von Faserstoffen zur Absenkung des pH-Wertes im Darm, um die Belastung durch Ammoniak zu vermindern (Umwandlungvon NH₃ zu NH₄⁺ und Ausscheidungvon NH₄⁺ über den Kot)

### Pankreatitis:

- Reduktion des Rohfettgehaltes
- Verwendung von hochverdaulichen Proteinquellen
- Verwendung von hochverdaulichen Kohlenhydratquellen

Welchen Vorteil haben die verwendeten Produkte/Rohstoffe nach der vorliegenden Patentanmeldung demgegenüber?
- Für Nieren-/Leber-/Pankreasdiäten sind besonders hoch verdauliche Proteinquellen notwendig. Bei einer pflanzlichen Fütterung eignen sich beispielsweise Proteinkonzentrate oder -isolate. Vielversprechend sind dabei vor allem Produkte aus pflanzlichen Quellen, die wenig sekundäre Pflanzenstoffe und/oder Rohfaser enthalten, da diese Komponenten die Verdaulichkeit beeinträchtigen können. Daher eignen sich insbesondere Konzentrate/Isolate aus "Nicht-Hülsenfrüchten" wie unter anderem aus Pilzen oder Bakterien/Hefen oder auch Einzelzellproteine (Single Cell Proteins).
- Zusätzlich erlaubt die fleischfreie Rezeptur, Hunde zu therapieren, die unter einer Futtermittelunverträglichkeit und zusätzlich unter einer weiteren Komorbidität leiden. Aktuell sind auf dem Markt kommerzielle Alleinfutter entweder für Futtermittelallergien oder für Nieren-/Leber-/Pankreaserkankungen verfügbar. Patienten mit multimorbiden Problematiken bleibt aktuell nur die Erstellung einer individuellen Ration, die von den Halterinnen selbstgekochtwerden muss. Die erfindungsgemäß verwendeten Rohstoffe erlauben das Erstellen von kommerziellen Alleinfuttern, die sowohl die Futtermittelallergie adressieren als auch Nieren-/Leber-/Pankreaserkrankungen.

### Literatur:

- Polzin, D.J. (2013), Evidence-based step-wise approach to managing chronic kidney disease in dogs and cats. Journal of Veterinary Emergency and Critical Care, 23: 205-215
- Polzin D. Chronic kidney disease. In: Ettinger S, Feldman E, editors. Textbook of veterinary internal medicine. Saunders; 2010. p. 2036-67.
- Jacob F, Polzin DJ, Osborne CA, et al. Clinical evaluation of dietary modification for treatment of spontaneous chronic renal failure in dogs. J Am Vet Med Assoc 2002;220(8): 1163-70.
- Finco D, Brown S, Crowell W, et al. Effects of dietary phosphorus and protein in dogs with chronic renal failure. Am J Vet Res 1992;53:2264-71.
- Haig, T. B. (1970). Experimental pancreatitis intensified by a high fat diet 914-918
- Lindsay, S., Entenman, C., & Chaikoff, I. L. (1948). Pancreatitis accompanying hepatic disease in dogs fed a high fat, low protein diet: 635-638.
- Moreno, A. A., Parker, V. J., Winston, J. A., & Rudinsky, A. J. (2022). Dietary fiber aids in the management of canine and feline gastrointestinal disease. Journal of the American Veterinary Medical Association, 260(S3), S33-S45.
- Mansfield, C. (2020). Pancreatitis in the Dog. Clinical Small Animal Internal Medicine, 591-600.
- Rebecca D. Norton, Catherine E. Lenox, Paul Manino, James C. Vulgamott; Nutritional Considerations for Dogs and Cats with Liver Disease. J Am Anim Hosp Assoc 1 January 2016; 52 (1): 1-7.
- Kamphues, J., Wolf, P., Coenen, M., Eder, K., Iben, C., Kienzle, E. & Zentek, J. (2014). Supplemente zur Tierernährung. 12. Auflage. M. & H. Scharper GmbH, Hannover, Deutschland.
- Meyer, H., & Zentek, J. (2010). Ernährung des Hundes: Grundlagen-Fütterung-Diätetik; 146 Tabellen. Georg Thieme Verlag.

### Hautproblematiken

- Juckreiz und in Folge Alopezie und bakterielle Sekundärinfektionen gehören zu den häufigsten Symptomatiken, unter denen Hunde mit Futtermittelallergien oder Umweltallergie leiden (Umwelt- und Futtermittelallergie treten häufig gemeinsam auf). Dabei spielt neben der mechanischen Reizung der Haut durch das Kratzen auch die bei Allergikern in Teilen beeinträchtigte Hautbarriere eine Rolle. Therapeutisch können dabei nachweislich Omega-3-Fettsäuren in hoher Dosierung die Hautbarriere unterstützen. Die therapeutisch wirksamen Omega-3-Fettäuren EPA&DHA (Eicosapentaensäure&Docosahexaensäure) finden sich für gewöhnlich in relevanter Konzentration nur in tierischen Rohstoffen, insbesondere Fischölen.
- Da Allergien beim Hund maßgeblich gegen tierische Proteine bestehen, gestaltet sich die Unterstützung der Hautbarriere bei diesen Pateinten mit Omega-3-Fettsäuren aus tierischen Rohstoffen teilweise schwierig.
- Abhilfe schaffen können hier Algenöle aus speziellen Algensorten, die EPA&DHA in ausreichend hoher Konzentration enthalten.

### Literatur:

- Olivry, T., & Mueller, R. S. (2019). Critically appraised topic on adverse food reactions of companion animals (7): signalment and cutaneous manifestations of dogs and cats with adverse food reactions. BMC veterinary research, 15, 1-6.
- Kaur, H., Singla, A., Singh, S., Shilwant, S., & Kaur, R. (2020). Role of omega-3 fatty acids in canine health: a review. International Journal of Current Microbiology and Applied Sciences, 9(3), 2283-2293.
- Logas, D., & Kunkle, G. A. (1994). Double-blinded crossover study with marine oil supplementation containing high-dose icosapentaenoic acid for the treatment of canine pruritic skin disease. Veterinary Dermatology, 5(3), 99-104.

### Chronisch-entzündliche Darmerkrankungen

- Definition: Symptomatik > 3 Wochen anhaltend
- Ursachen vielfältig, beispielsweise immunmediiert (z.B. IBD, Inflammatory Bowel Disease) oder futtermittelassoziiert.

Die konventionelle Einteilung bisher:
- Futtermittelresponsiv (50-60 %)
- Antibiotika-responsiv (15-35 %)
- Immunsuppression-responsiv (10-25 %)
- Nicht-responsiv (5-45 %)

Diese Einteilung wird mittlerweile als überholt beurteilt, da in den letzten Jahren die Rolle der Diätetik weiter erforscht wurde. Es zeigte sich, dass selbst bei den zuvor als "nicht-responsiv" eingeordneten chronischen Darmerkrankungen bei bis zu 68 % der Fälle eine Besserung eintritt, wenn eine passende Diät verwendet wird. Dies hängt maßgeblich mit der Bedeutung des gastrointestinalen Mikrobioms zusammen, das bei allen chronisch-entzündlichen Darmerkrankungen eine Rolle zu spielen scheint, unabhängig von der Pathogenese. Aus diesem Grund wird der Diät bei chronischen Darmpatienten mittlerweile eine deutlich größere Bedeutung beigemessen. Es wird davon ausgegangen, dass der Großteil der Patienten von einer Anpassung der Diät profitieren kann. Für chronische Darmpatienten ist eine gut verdauliche, für Allergiker geeignete Diät daher bedeutend. Futtermittel mit gut verdaulichen alternativen Proteinquellen, beispielsweise Proteinkonzentrate/-isolate aus Pflanzen, Pilzen, Bakterien oder Hefen (Single Cell Protein), sind daher eine gute Option für diese Patienten.

### Literatur:

- Dupouy-Manescau, N., Méric, T., Sénécat, O., Drut, A., Valentin, S., Leal, R. O., & Hernandez, J. (2024). Updating the classification of chronic inflammatory enteropathies in dogs. Animals, 14(5), 681. Dandrieux, J. R. S. (2016). Inflammatory bowel disease versus chronic enteropathy in dogs: are they one and the same? Journal of Small Animal Practice, 57(11), 589-599.
- Dandrieux, J. R. S., & Mansfield, C. S. (2019). Chronic enteropathy in canines: prevalence, impact and management strategies. Veterinary Medicine: Research and Reports, 203-214*.*
- Allenspach, K., Wieland, B., Gröne, A., & Gaschen, F. (2007). Chronic enteropathies in dogs: evaluation of risk factors for negative outcome. Journal of veterinary internal medicine, 21(4), 700-708.
- Allenspach, K., Culverwell, C., & Chan, D. (2016). Long-term outcome in dogs with chronic enteropathies: 203 cases. Vet Rec, 178(15), 368.

Futtermittelallergien können also wie oben diskutiert gegen eine Vielzahl von Inhaltsstoffen in Tierfutter bestehen, darunter am häufigsten tierische Proteine wie Rindfleisch, Huhn, Lamm oder Fisch. In manchen Fällen können auch Allergien gegen pflanzliche Proteinquellen wie Weizen, Mais oder Soja bestehen.

Die Symptome von Futtermittelallergien bei Tieren können wie oben geschildert variieren, aber zu den häufigsten gehören: Hautprobleme wie Juckreiz, Hautausschläge, Hautentzündungen und Haarausfall. Gastrointestinale Probleme wie Erbrechen, Durchfall, Blähungen und Bauchschmerzen können teilweise ebenfalls auftreten.

Die Überempfindlichkeitsreaktion des Immunsystems von Tieren auf bestimmte Bestandteile im Futter kann zudem wie oben diskutiert Bestandteil von (chronischen) entzündlichen Darmerkrankungen ("inflammatory bowel diseases"-IBD) sein.

Die Behandlung von Futtermittelallergien bei Tieren beinhaltet in der Regel die Vermeidung der auslösenden Nahrungsmittel durch die Umstellung auf einspeziell allergenarmes (hypoallergene) Futtermittel. Es können auch Medikamente wie corticosteroidhaltige Präparate zur Linderung von Symptomen eingesetzt werden.

Es ist möglich, die Symptome von Futtermittelallergien bei z.B. von Natur aus fleischfressenden Tieren wie Hunden und Katzen durch die Verwendung von veganen Futtermitteln zu lindern, da dies bestimmte häufige Allergene wie tierische Proteine ausschließt. Eine Voraussetzung ist dabei aber, dass das Tier nicht bereits auf bestimmte pflanzliche Inhaltsstoffe, wie sie z.B. in Soja, Weizen, Mais, Hülsenfrüchten enthalten sind, allergisch reagiert.

Die pflanzliche Ernährung von Hunden und Katzen kann zudem einige potenzielle Nachteile haben, die berücksichtigt werden müssen:
Nährstoffmängel: Hunde und Katzen sind von Natur aus Fleischfresser und benötigen eine Vielzahl von Nährstoffen, die normalerweise in tierischen Produkten wie Fleisch, Fisch und Innereien vorkommen. Pflanzliche Ernährung kann zu Mängeln an bestimmten Nährstoffen wie Protein, Omega-3-Fettsäuren, Vitamin B12, Eisen, Zink und Aminosäuren führen, es sei denn, sie werden durch geeignete Ergänzungen ausgeglichen.

Verdaulichkeit: Pflanzliche Proteine sind für Hunde und Katzen oft weniger leicht verdaulich als tierische Proteine, so dass die Tiere Schwierigkeiten haben können, alle benötigten Nährstoffe aus pflanzlicher Nahrung zu absorbieren, was zu Verdauungsproblemen wie Blähungen, Durchfall und Magenbeschwerden führen kann.

Biologische Verfügbarkeit von Nährstoffen: Einige Nährstoffe in pflanzlichen Lebensmitteln sind möglicherweise in einer Form vorhanden, die für Hunde und Katzen weniger biologisch verfügbar ist als in tierischen Produkten. Dies bedeutet, dass die Tiere möglicherweise größere Mengen an pflanzlicher Nahrung benötigen, um ihren Nährstoffbedarf zu decken.

Geschmack und Vorlieben: Viele Hunde und Katzen bevorzugen den Geschmack von Fleisch und tierischen Produkten gegenüber pflanzlichen Futtermitteln. Es kann schwierig sein, die Tiere dazu zu bringen, pflanzliche Diäten zu akzeptieren, insbesondere wenn sie nicht ausreichend an diese Art von Ernährung gewöhnt sind.

Die Aufgabe der Erfindung ist daher die Bereitstellung einer veganen Tierfuttermittelzusammensetzung, die die obigen ernährungsphysiologischen Anforderungen zufriedenstellend erfüllt und zur Behandlung von Futtermittelallergien und entzündlichen Darmerkrankungen sowie Komorbiditäten bei Tieren verwendet werden kann.

Erfindungsgemäß wird diese Aufgabe gelöst durch eine vegane Tierfuttermittelzusammensetzung zur Verwendung zur Behandlung von Futtermittelallergien, entzündlichen Darmerkrankungen, Nierenerkrankungen, Lebererkrankungen, Bauchspeicheldrüsenerkrankungen, Hauterkrankungen und Übergewicht bei Tieren sowie zur purinarmen Ernährung von Tieren, wobei die vegane Futtermittelzusammensetzung enthält :
5 bis 60 Gew.-% bezogen auf das Trockengewicht der Zusammensetzung eines Einzelzellproteinprodukts aus der Biomasse einer Mikroorganismusspezies;
0,5 bis 65 Gew.-% bezogen auf das Trockengewicht der Zusammensetzung Kohlenhydrate nicht-tierischen Ursprungs;
0.5 bis 10 Gew.-% bezogen auf das Trockengewicht der Zusammensetzung Fette nicht-tierischen Ursprungs;
0,001 bis 20 Gew.-% bezogen auf das Trockengewicht der Zusammensetzung pflanzliche Faserstoffe.

Beispiele für mit der erfindungsgemäß verwendeten Tierfuttermittelzusammensetzung behandelbare Nieren-, Leber-, Bauchspeicheldrüsen- und Hauterkrankungen sind:
Leber:
   - Hepatitis (entzündliche Lebererkrankung)
   - hepatische Lipidose (=Leberverfettung-vor allem bei der Katze sehr relevant)
   - Leberzirrhose (Endstadium einer chronischen Lebererkrankung)
Niere:
   - Chronische Nierenerkrankung, z.B. Glomerulonephritis (Entzündung der kleinen Filtereinheiten der Niere (Glomeruli). Vor allem bei Katzen, aber auch Hunden häufig. Bei Katzen: Haupttodesursache bei geriatrischen Katzen. Nierendiät ist die wichtigste Therapie
   - akute Nierenerkrankung (auch hier Nierendiät nötig)
Bauchspeicheldrüse:
   - Pankreatitis (Bauchspeicheldrüsenentzündung-Diät ist hier die wichtigste Therapie)
   - exokrine Pankreasinsuffizienz (Mangel an Verdauungsenzymen-Diät ist hier ebenfalls von großer Bedeutung)
Hautprobleme:
   - Viele Futtermittelallergiker leiden unter chronischem Juckreiz und einer gestörten Hautbarriere. Dadurch können Sekundärinfektionen mit Bakterien und Pilzen entstehen
   - weitere Hautprobleme wie Entzündungen und Juckreiz

Die erfindungsgemäß verwendete Tierfuttermittelzusammensetzung weist folgende Vorteile auf:
1. Vegan
2. leichte und kostengünstige Herstellbarkeit in großen Mengen
3. keine Aufreinigung erforderlich
4. alle essenziellenAminosäuredichte
5. leichte Einstellbarkeit von gewünschten Aminosäureprofilen bzw. -mustern hoher natürlicher Glutamatgehalt (mindestens 5 Gew.-% bis etwa 15 Gew.-%)
6. hohe Verdaulichkeit (ca. 90% und höher)
7. geringe Allergenität
8. keine antinutritiven sekundären Pflanzeninhaltsstoffe enthalten
9. guter Geschmack und guter Geruch
10. gute Akzeptanz durch die Tiere
11. hohe Lagerstabilität
12. keine anderen Proteinquellen erforderlich, aber optionale Kombination mit pflanzlichen Proteinen möglich
13. Verwendung als Alleinfutter möglich

Vorteilhafte und/oder bevorzugte Ausführungsformen der Erfindung sind Gegenstand der Unteransprüche.

Im Folgenden wird die Erfindung detailliert beschrieben, wobei die offenbarten konkreten Ausführungsformen der Erfindung, Beispiele oder Ergebnisse lediglich zur Veranschaulichung dienen sollen und keinesfalls als Einschränkung des Schutzbereichs der Erfindung, wie dieser in den beigefügten Ansprüchen definiert ist, zu interpretieren sind.

Sofern nicht anders definiert, haben alle hier verwendeten technischen und wissenschaftlichen Begriffe die gleiche Bedeutung, wie sie von einem Fachmann auf dem technischen Gebiet der Erfindung verstanden werden. Dabei kann der Fachmann auch auf die einleitenden Erläuterungen vollumfänglich Bezug nehmen.

Die Verwendung von bestimmten bzw. unbestimmten Artikeln ("der", "die", "das", "ein", "eine"), ist (insbesondere im Zusammenhang mit den Ansprüchen) so zu verstehen, dass davon mindestens ein Element bzw. eine Komponente umfasst sein soll, sofern hier nichts anderes angegeben ist oder sich durch den Kontext eindeutig etwas anderes ergibt.

Der Konjunktion "oder" ist als einschließendes und nicht als ausschließendes "oder" zu verstehen, d. h. als "und/oder", sofern hier nichts anderes angegeben ist oder sich durch den Kontext eindeutig etwas anderes ergibt.

Die Verwendung von Begriffen wie "zum Beispiel", "z.B.", "wie" oder Abwandlungen davon soll lediglich zur besseren Veranschaulichung der Erfindung dienen und darf keinesfalls als Einschränkung des Schutzbereichs der Erfindung, wie er in den beigefügten Ansprüchen definiert ist, interpretiert werden.

Sämtliche numerischen Werte verstehen sich, unabhängig davon, ob dies ausdrücklich angegeben ist oder nicht, als "ungefähre" Werte (zumindest im Rahmen der üblichen Fehlertoleranzen). Die Angabe von Wertebereichen dient zudem lediglich zur Abkürzung und bezieht sich, sofern hier nicht anderes angegeben ist, auf jeden einzelnen Wert, der in den Bereich fällt, auch wenn dieser Wert nicht individuell angegeben wird.

Tierfuttermittel im Sinne der Erfindung sind speziell formulierte Futter, die zur Ernährung von Nutztieren und Haus- bzw. Heimtieren verwendet werden. Sie sind darauf ausgerichtet, die ernährungsphysiologischen Bedürfnisse verschiedener Tierarten zu erfüllen und ihnen alle erforderlichen Nährstoffe in angemessenen Mengen bereitzustellen, um ihre Gesundheit und ihr Wohlbefinden zu erhalten. Tierfuttermittel können in verschiedenen Formen vorliegen, darunter Trockenfutter, Nassfutter, Halbfeuchtfutter und Snacks. Sie enthalten eine Mischung aus Proteinen, Kohlenhydraten, Fetten, Vitaminen, Mineralstoffen und anderen Nährstoffen, die von dem jeweiligen Tier benötigt werden. Die Zusammensetzung von Tierfuttermitteln kann je nach Tierart, Alter, Aktivitätsniveau, Gesundheitszustand und anderen individuellen Faktoren variieren. Zum Beispiel benötigen junge Tiere möglicherweise mehr Protein und Kalorien für ihr Wachstum, während ältere Tiere möglicherweise eine spezielle Ernährung benötigen, um altersbedingte Gesundheitsprobleme zu verhindern oder zu behandeln. Tierfuttermittel werden nach strengen gesetzlichen Vorschriften hergestellt und müssen die Ernährungsstandards erfüllen, die von Behörden wie der FDA (Food and Drug Administration, USA) oder vergleichbaren Institutionen wie der FEDIAF (European Pet Food Industry Federation) in anderen Ländern festgelegt werden. Diese Standards sollen sicherstellen, dass Tierfuttermittel sicher, qualitativ hochwertig und ernährungsphysiologisch angemessen sind.

Der Begriff "vegan" im Sinne der Erfindung bezieht sich auf eine Ernährungsweise, die darauf abzielt, alle Tierprodukte zu meiden und auf tierische Bestandteile in Nahrungsmitteln zu verzichten. Stattdessen werden pflanzliche Produkte zur Ernährung genutzt. Der Begriff "vegan" soll hier den Begriff "vegetarisch" mitumfassen.

Der Begriff "Biomasse" von Mikroorganismen (z.B. Bakterien, Hefen, Pilze, Algen) im Sinne der Erfindung bezieht sich auf die Menge an organischem Material, das von einer Population von Mikroorganismen in einem Habitat, hier in einem Bioreaktor oder Fermenter, gebildet wird. Die Biomasse von Mikroorganismen kann abhängig von Faktoren wie Nährstoffverfügbarkeit, Temperatur, Feuchtigkeit, pH-Wert und anderen Umweltbedingungen variieren.

Halophile Bakterien und Pilze im Sinne der Erfindung sind Organismen, die in der Lage sind, in salzhaltigen Umgebungen zu leben und zu wachsen. Diese Organismen haben sich an extreme Salzgehalte (insbesondere NaCl, bis etwa 5 M) angepasst, die für die meisten anderen Lebensformen tödlich wären. Halophile Bakterien können in hochsalzigen Habitaten wie Salzseen, Salzpfannen, Salzkrusten oder Salzminen gefunden werden. Sie haben Mechanismen entwickelt, um mit dem hohen Salzgehalt in ihrer Umgebung umzugehen, wie z.B. die Synthese von osmotisch aktiven Stoffen, um den Wasserhaushalt zu regulieren, oder die Anpassung ihrer Zellwände, um den osmotischen Druck auszugleichen.

Thermophile Bakterien und Pilze im Sinne der Erfindung sind Organismen, die in der Lage sind, unter extremen Hitzebedingungen zu leben und zu gedeihen. Thermophile Bakterien und Pilze bevorzugen Temperaturen im Bereich von etwa 45°C bis über 80°C und können in Umgebungen wie z.B. heißen Quellen und Geysiren vorkommen. Diese Bakterien und Pilze haben sich an ihre extremen Lebensräume angepasst und verfügen über spezielle Enzyme, Proteine und Membranlipide, die hitzebeständig sind und ihnen helfen, ihre Zellstrukturen und Funktionen bei hohen Temperaturen aufrechtzuerhalten. Einige thermophile Bakterien und Pilze können sogar in Umgebungen mit Temperaturen über dem Siedepunkt von Wasser (über 120 °C) leben, indem sie spezielle Mechanismen zur Stabilisierung ihrer Zellen und zur Verhinderung der Denaturierung ihrer Proteine entwickeln.

Der Begriff "Protein" oder "Proteine", wie er hier verwendet wird, umfasst Proteine, Peptide und Polypeptide, wobei diese Proteine, Peptide oder Polypeptide posttranslational modifiziert sein können oder nicht. Eine posttranslationale Modifikation kann zum Beispiel eine Phosphorylierung, Methylierung oder Glykosylierung sein. Der Begriff "Protein" wird auch in Bezug auf eine Fraktion oder eine Zusammensetzung verwendet, die Proteine, wie hier definiert, enthält. Durch enzymatischer Behandlung mit Protease(n), d. h. Enzymen, die die Hydrolyse von Peptidbindungen katalysieren können, werden Peptidbindungen in Proteinen hydrolysiert und größere Polypeptide in kleinere Polypeptide und/oder Aminosäuren umgewandelt. Der Begriff "Aminosäuren und Peptide" bezieht sich somit auch auf eine Zusammensetzung, die Proteine, Peptide (die als Polypeptide oder Oligopeptide bezeichnet werden können) und Aminosäuren enthält.

Der Begriff "Einzelzellprotein" (single-cell protein"-SCP) im Sinne der Erfindung bezieht sich auf Proteine, die in der Biomasse von Zellen eines Typs von Mikroorganismen wie Bakterien, Algen, Pilzen oder Hefen gewonnen werden. Die Mikroorganismen werden in großen Mengen kultiviert und können so als Nahrungsquelle für Menschen, Tiere oder andere Zwecke verwendet werden. SCP hat das Potenzial, eine nachhaltige Proteinquelle bereitzustellen, insbesondere angesichts der steigenden Nachfrage nach Protein bei der wachsenden Weltbevölkerung und der begrenzten Verfügbarkeit von landwirtschaftlichen Flächen für den Anbau von Nutzpflanzen und Viehfutter. Im Folgenden seien einige Eigenschaften und Merkmale von SCP genannt:
1. Hoher Proteingehalt: SCP weist einen hohen Proteingehalt auf, der dem von traditionellen Proteinquellen wie Fleisch, Fisch oder Soja ähnlich sein kann. Dies macht SCP zu einer potenziellen Proteinquelle für die menschliche Ernährung oder die Tierfütterung.
2. Effiziente Produktion: Mikroorganismen wie Bakterien, Algen, Pilze oder Hefen können unter kontrollierten Bedingungen effizient kultiviert werden, was zu einer schnellen Produktion von SCP führt. Dies ermöglicht eine kontinuierliche Produktion unabhängig von klimatischen Bedingungen oder saisonalen Schwankungen.
3. Vielseitige Anwendungsmöglichkeiten: SCP kann für verschiedene Zwecke verwendet werden, z.B. zur menschlichen Ernährung und Tierfütterung, für Aquakulturen, in der Biotechnologie und zur Herstellung von Nahrungsergänzungsmitteln oder funktionellen Lebensmitteln.
4. Nachhaltigkeit: Die Produktion von SCP kann auf ressourceneffiziente Weise erfolgen und benötigt im Vergleich zu traditionellen Tierproteinquellen wie Viehzucht weniger Wasser, Land und Energie.

Antinutritive sekundäre Pflanzeninhaltsstoffe im Sinne der Erfindung sind natürliche chemische Verbindungen, die in Pflanzen vorkommen und die Aufnahme oder Verwertung von Nährstoffen im Körper beeinträchtigen können. Diese Verbindungen dienen oft als Abwehrmechanismus der Pflanzen gegen Schädlinge, Insekten oder andere Fressfeinde und können auch dazu beitragen, die Pflanze vor Krankheiten zu schützen. Während sie für die Pflanze nützlich sind, können sie bei Tieren oder Menschen, die diese Pflanzen verzehren, unerwünschte Effekte haben. Beispiele für antinutritive sekundäre Pflanzeninhaltsstoffe sind Phytinsäure, Lecithine, Solanin, Oxalate und Tannine.

Entzündliche Darmerkrankungen (z.B. "inflammatory bowel disease"-IBD) im Sinne der Erfindung ist ein Sammelbegriff für eine Gruppe von chronischen Darmerkrankungen, die durch eine Entzündung der Darmwand gekennzeichnet sind. IBD können auch bei Tieren, einschließlich Hunden und Katzen, auftreten. Ein Beispiel ist die chronische enteropathische Enteritis bei Hunden. Diese Form von IBD betrifft den Dünndarm von Hunden. Sie wird oft durch eine übermäßige Immunantwort auf bestimmte Nahrungsmittelbestandteile, Umweltfaktoren oder Bakterien verursacht. Symptome können Durchfall, Erbrechen, Gewichtsverlust, Appetitlosigkeit und abdominale Schmerzen umfassen. Die genauen Ursachen von IBD bei Tieren sind oft unbekannt, aber Faktoren wie genetische Veranlagung, Umweltfaktoren, Ernährung, Darmdysbiose (Ungleichgewicht der Darmflora) und Immunreaktionen spielen eine Rolle. Die Behandlung von IBD bei Tieren umfasst in der Regel eine Kombination aus medizinischer Therapie und Diätmaßnahmen. Dazu gehören die Verabreichung von entzündungshemmenden Medikamenten wie Kortikosteroiden oder Immunsuppressiva, die Verwendung von speziellen Diäten (z.B. hypoallergene Diäten oder Diäten mit leicht verdaulichen Inhaltsstoffen), die Kontrolle von Begleiterkrankungen (Komorbiditäten) wie Nierenerkrankungen, Lebererkrankungen, Bauchspeicheldrüsenerkrankungen, Hauterkrankungen und die Vermeidung von Auslösern, die die Symptome verschlimmern könnten.

Eine purinarme Ernährung im Sinne der Erfindung ist eine Ernährungsweise, bei der Lebensmittel vermieden oder in begrenztem Maße konsumiert werden, die reich an Purinen sind. Purine sind natürliche Bestandteile von Nucleinsäuren.Beim Abbau von Nahrungs-Purinen entsteht im Körper als Zwischenprodukt Harnsäure. Bei gesunden Tieren wird die Harnsäure in Allantoin umgewandelt und über die Niere ausgeschieden. Dalmatinern fehlt aus genetischen Gründen ein Enzym (Urikase), das Harnsäure in Allantoin umwandelt. Dadurch häuft sich die Harnsäure bei Dalmatinern an, es besteht die Gefahr der Bildung von Uratsteinen in der Harnblase. Dalmatiner sollten aus diesem Grund purinarm ernährt werden. Alternativ können auch Hunde anderer Rassen (das heißt nicht Dalmatiner) von Hyperurikämie betroffen sein, beispielsweise aus genetischen Gründen. Diese Hunde können ebenfalls eine purinarme Diät im Sinne der vorliegenden Erfindung erhalten.

Bei Katzen stellen Uratsteine ebenfalls einen relevanten Anteil der auftretenden Harnsteintypen, so dass auch hier eine purinarme Diät notwendig ist. Eine purinarme Diät ist auch erforderlich zur Prophylaxe von Harnsteinbildung bei Hunden, die an Leishmaniose erkrankt sind und mit Allopurinol behandelt werden, da Allopurinol den Abbau von Purinen zu Harnsäure durch Hemmung des Enzyms Xanthinoxidase unterbindet.

Essentielle Mineralstoffe im Sinne der Erfindung, auch bekannt als Mineralstoffe oder Mineralien, sind anorganische Elemente, die der Körper für eine Vielzahl von lebenswichtigen Funktionen benötigt. Diese Mineralstoffe sind "essentiell", weil der Körper sie nicht selbst produzieren kann und sie daher in ausgewogenen Mengen über die Nahrung aufgenommen werden müssen. Sie spielen eine wichtige Rolle bei der Aufrechterhaltung der Gesundheit und des Wohlbefindens, da sie an zahlreichen physiologischen Prozessen beteiligt sind. Einige der wichtigsten essentiellen Mineralstoffe sind die Mengenelemente Calcium, Magnesium, Kalium, Natrium, Phosphor.

Spurenelemente im Sinne der Erfindung sind essentielle Mineralstoffe, die der Körper in sehr kleinen Mengen benötigt, aber die dennoch lebenswichtig sind. Dazu gehören beispielsweise Eisen, Zink, Kupfer, Jod, Selen und Mangan. Obwohl sie nur in Spuren im Körper vorkommen, spielen sie eine wichtige Rolle bei zahlreichen biologischen Prozessen, wie zum Beispiel dem Stoffwechsel, der Immunfunktion und der Zellentwicklung. Eine unzureichende Zufuhr von Spurenelementen kann zu Mangelerscheinungen und gesundheitlichen Problemen führen, während ein angemessener Verzehr zur Erhaltung der Gesundheit beiträgt.

Essentielle Vitamine im Sinne der Erfindung sind organische Verbindungen, die der Körper benötigt, um normale physiologische Funktionen aufrechtzuerhalten. Diese Vitamine müssen über die Ernährung aufgenommen werden, da sie lebensnotwendig sind und der Körper sie nicht selbst herstellen kann. Vitamine spielen eine wichtige Rolle bei der Regulierung des Stoffwechsels, der Zellfunktionen, des Wachstums und der Entwicklung sowie der Erhaltung der Gesundheit. Beispiele für essentielle Vitamine sind die fettlöslichen Vitamine E (Tocopherol), D, K, A (Retinol), die wasserlöslichen Vitamine B1 (Thiamin), B2 (Riboflavin), B3 (Niacin), B5 (Pantothensäure), B6 (Pyridoxin), B7 (Biotin), B9 (Folsäure) und B12 (Cobalamin).

Die erfindungsgemäß zur Behandlung von Futtermittelallergien und entzündlichen Darmerkrankungen sowie damit assoziierten Komorbiditäten bei Tieren, z.B. Nutz- und Heimtieren (insbesondere Hunde und Katzen) verwendete vegane Tierfuttermittelzusammensetzung enthält ein Einzelzellproteinprodukt aus der Biomasse eines Typs einer Mikroorganismusspezies. Die Mikroorganismusspezies kann z.B. unter Bakterienspezies, Hefespezies, Pilzspezies, Algenspezies und Protozoen ausgewählt werden. Es ist klar, dass nur solche Mikroorganismen gewählt werden, die keine unverträglichen, schädlichen oder giftigen Substanzen produzieren (wie etwa bestimmte Bakterien- oder Pilztoxine). Vorteilhafterweise können halophile und/oder thermophile Bakterien- und Pilzspezies verwendet werden, da diese bei für andere Mikroorganismen tödlich hohen Salzkonzentrationen und Temperaturen wachsen können. Dadurch wird das Kontaminationsrisiko durch andere Mikroorganismen reduziert, so dass unter nicht-sterilen Bedingungen gearbeitet werden kann, was erhebliche Energiekosten für die thermische Sterilisierung von z.B. Nährmedien und Fermentern einspart. Außerdem kann bei der Verwendung von halophilen Mikroorganismen salziges Meerwasser für die zur industriellen Kultivierung in großen Mengen erforderlichen Nährmedien eingesetzt werden, was erhebliche Kostenvorteile hat.

Die Bakterienspezies kann z.B. aus der aus den folgenden Bakteriengattungen bestehenden Gruppe ausgewählt werden: Vibrio, Geobacillus, Bacillus, Cupriavidus, Lactococcus, Lactobacillus, Enterococcus, Streptococcus, und Pediococcus. Konkrete Spezies sind z.B. Vibrio natriegens, Geobacillus LC300, Bacillus megaterium, Cupriavidus necator DSM 531 und Cupriavidus necator DSM 541. Auch gentechnisch veränderte Mutanten davon können eingesetzt werden.

Die Pilzspezies kann z.B. aus der aus den folgenden Pilzgattungen bestehenden Gruppe ausgewählt werden: Fusarium, Rhizopus, Aspergillus, Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Mvriococcum, Corynascus und Coonemeria. Konkrete Spezies sind z.B. Fusarium venenatum, Rhizopus oligosporus, Rhizopus oryzae, Aspergillus oryzae, Aspergillus sojae, Rhizomucor pusillus und Rhizomucor pusillus CBS 143028. Auch gentechnisch veränderte Mutanten davon können eingesetzt werden.

Neben Biomasse aus den oben beispielhaft genannten natürlich vorkommenden Bakterien- und Pilzspezies kann auch Biomasse aus genetisch veränderten (rekombinanten) Bakterien und Pilzen als Quelle für Einzelzellproteinprodukte verwendet werden. Der verwendete gentechnisch veränderte Organismus unterliegt keinen besonderen Beschränkungen und kann eine genetische Veränderung zur Verbesserung der Wachstumsrate, des Biomasseertrags und/oder der Produktion bestimmter chemischer Substanzen, z.B. eines gewünschten Aminosäureprofils oder -musters, umfassen. Mit Hilfe der rekombinanten DNA-Technologie können z.B. mutierte Gene isoliert werden, die hohe Mengen an spezifischen Aminosäuren wie Glutamat, Tryptophan und Phenylalanin sowie hohe Proteinausbeuten produzieren können. Gewünschte Aminosäureprofile oder -muster lassen sich aber auch z.B. einfach durch Zusammenmischen verschiedener Einzelzellproteinprodukte aus unterschiedlichen Biomassen von unterschiedlichen Mikroorganismen herstellen.

Die in der erfindungsgemäßen verwendeten veganen Tierfuttermittelzusammensetzung eingesetzte Biomasse ist mit üblichen Kultivierungsverfahren erhältlich. Besonders vorteilhaft ist das in der WO 2022/268842 A1 von MikroHarvest GmbH beschriebene Kultivierungsverfahren, da es die Produktion großer Mengen Biomasse innerhalb kurzer Zeit gestattet. Kurz zusammengefasst wird bei diesem Verfahren ein Mikroorganismusstamm mit einer sehr hohen Wachstumsrate (von mindestens 0.85 h⁻¹), z.B. Vibrio natriegens, Geobacillus LC300, Bacillus megaterium, in einem Nährmedium in einem Fermenter im Industriemaßstab kultiviert.

Nach Beendigung der Kultivierung wird die Biomasse nach dem Fachmann bekannten Verfahren geerntet. Beispielsweise werden zur Abtrennung der Biomasse vom Nährmedium die Tangentialstromfiltration oder industrielle Düsen- oder Zentrifugalseparatoren eingesetzt. Die so erhaltene Biomasse hat eine Trockenmasse zwischen 10 und 25 Gew.-%. Anschließen werden die Zellen der Biomasse nach bekannten Verfahren mechanisch, z.B. mit einer Kugelmühle oder durch Anwendung von gepulsten elektrischen Feldern (PEF), thermische Autolyse oder chemisch, z.B. durch Behandlung mit Salzen, basischen Reagenzien und/oder Tensiden, aufgeschlossen (desintegriert, lysiert). Das so erhaltene Produkt enthält mindestens 40% Einzelzellprotein. Das Einzelzellprotein kann optional mit bekannten Verfahren, z.B. durch enzymatische Behandlung mit Protease(n) unter Erhalt von Aminosäuren und Peptiden hydrolysiert werden.

Das in der erfindungsgemäß verwendeten veganen Tierfuttermittelzusammensetzung enthaltene Einzelzellproteinprodukt aus der Biomasse einer Mikroorganismusspezies kann in beliebiger Form vorliegen, z.B. kann es sich um ein Zelllysat, Proteinkonzentrat, Proteinisolat, Proteinextrakt oder Proteinhydrolysat handeln und/oder um freie Aminosäuren, Peptide und Oligopeptide und Kombinationen daraus. Der Fachmann kann die Form des Einzelzellproteinprodukts nach Maßgabe der praktischen Gegebenheiten wählen, besondere Beschränkungen gibt es dabei nicht.

Optional können den erfindungsgemäß verwendeten Einzelzellproteinprodukten auch pflanzliches Protein zugesetzt werden.

Die in der erfindungsgemäß verwendeten veganen Tierfuttermittelzusammensetzung enthaltenen nicht-tierischen Kohlenhydrate unterliegen keinen besonderen Beschränkungen und können z.B. aus der aus Buchweizenkohlenhydrat, Dinkelkohlenhydrat, Amaranthkohlenhydrat, Quinoakohlenhydrat, Weizenkohlenhydrat, Hirsenkohlenhydrat, Süßkartoffelkohlenhydrat, Maniokkohlenhydrat, Roggenkohlenhydrat, Gerstenkohlenhydrat, Haferkohlenhydrat, Maiskohlenhydrat, Reiskohlenhydrat, Kartoffelkohlenhydrat und Kombinationen davon bestehenden Gruppe ausgewählt werden.

Die in der erfindungsgemäß verwendeten veganen Tierfuttermittelzusammensetzung enthaltenen Fette unterliegen keinen besonderen Beschränkungen und können z.B. aus der aus Sonnenblumenöl, Algenöl, Chiasamenöl, Sesamöl, Nachtkerzenöl, Kürbiskernöl, Traubenkernöl, Sanddornöl, Hagebuttenkernöl, Arganöl, Schwarzkümmelöl, Borretschöl, Aprikosenkernöl, Mandelöl, Erdnussöl, Leinöl, Leinsamenschrot, Leindotteröl, Olivenöl, Rapsöl, Maiskeimöl, Haselnussöl, Hanföl, Reiskeimöl, Sesamöl, Distelöl, Sojaöl, Palmöl, Kokosfett, Wallnussöl oder Kombinationen davon bestehenden Gruppe ausgewählt werden.

Die in der erfindungsgemäß verwendeten veganen Tierfuttermittelzusammensetzung enthaltenen pflanzlichen Faserstoffe unterliegen keinen besonderen Beschränkungen und können z.B. unter Cellulose, Pektin, Hemicellulose und Lignin ausgewählt werden.

Die erfindungsgemäß verwendete vegane Tierfuttermittelzusammensetzung kann zusätzlich Mengenelemente, Spurenelemente und Vitamine enthalten. Beispiele für Mengenelemente sind Natrium, Kalium, Calcium, Magnesium, Chlorid, Phosphat, Sulfat und Kombinationen daraus. Beispiele für Spurenelemente sind Eisen, Jod, Zink, Selen, Kupfer, Fluorid und Kombinationen daraus. Beispiele für Vitamine sind die fettlöslichen Vitaminen E, D, K, A, die wasserlöslichen Vitamin B1 (Thiamin), Vitamin B2 (Riboflavin), Vitamin B3 (Niacin), Vitamin B5 (Pantothensäure), Vitamin B6 (Pyridoxin), Vitamin B7 (Biotin), Vitamin B9 (Folsäure), Vitamin B12 (Cobalamin).

Die erfindungsgemäß verwendete vegane Tierfuttermittelzusammensetzung kann in beliebiger Form eingesetzt werden, z.B. in Form von Trockenfutter ("Snacks") oder Nassfutter.

Die Verdaulichkeit und Schmackhaftigkeit der erfindungsgemäß verwendeten veganen Tierfuttermittelzusammensetzung ist sehr gut. Verdaulichkeit und Schmackhaftigkeit sowie andere ernährungsphysiologische Parameter wie die Proteinqualität, Aminosäuren-bioverfügbarkeit, Aminosäurenindex etc. können wie in der EP 3 909 435 A1 der Anmelderin beschrieben bestimmt und berechnet werden.

Nachstehend einige Beispiele für die Zusammensetzung erfindungsgemäß verwendeter Tierfuttermittelzusammensetzungen:

**Tabelle 1**

| | **Beispiel 1** | **Beispiel 2** | **Beispiel 3** |
|---|---|---|---|
| | % | % | % |
| Einzelzellprotein (Single Cell Protein, SCP) | 26 | 16 | 50 |
| Pflanzenprotein | / | 15 | / |
| Kohlenhydrate | 56 | 50 | 32 |
| Gemüse/Obst | 12 | 12 | 12 |
| Fette/Öle | 2 | 3 | 2 |
| Mineralien/Vitamine | 4 | 4 | 4 |
| Summe | 100 | 100 | 100 |

Nachstehend ein Beispiel für ein Aminosäureprofil einer erfindungsgemäß verwendeten Tierfuttermittelzusammensetzung im Vergleich zu Soja, einer Referenzproteinquelle im pflanzlichen Bereich, da Soja ein vollständiges Aminosäureprofil, d.h.mit sämtlichen essenziellen Aminosäuren, aufweist:

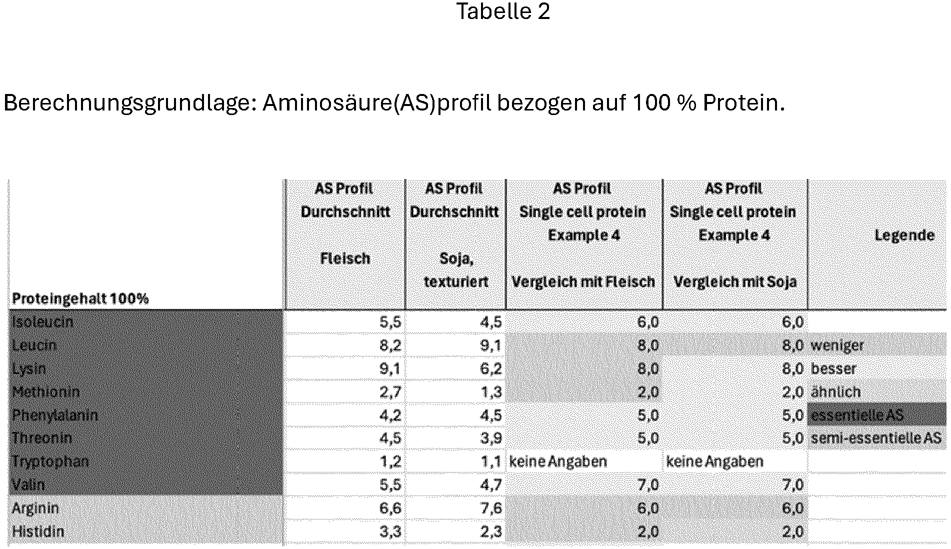

Als essentielle Aminosäuren sind genannt: Isoleucin, Leucin, Lysin, Methionin, Phenylalanin, Threonin, Tryptophan, Valin.

Als semi-essentielle Aminosäuren sind genannt: Arginin, Histidin.

Die Tabelle zeigt, dass das erfindungsgemäß verwendete Einzelzellprotein (Single Cell Protein, SCP) Im Vergleich zu Soja bzgl. des Aminosäureprofils sehr gut abschneidet. Auch im Vergleich zu Fleisch schneidet das SCP gut ab. Zum Vergleich wurde ein durchschnittliches Aminosäureprofilvon Hühner- und Rindfleisch herangezogen.

Fachleuten auf dem technischen Gebiet der Erfindung ist klar, dass die oben beschriebenen repräsentativen Ausführungsformen und Details der Erfindung nur zur Veranschaulichung der vorliegenden Erfindung dienen sollen, und dass verschiedene Abänderungen und Modifikationen vorgenommen werden können, ohne dadurch den Schutzbereichs der Erfindung, wie er in den beigefügten Ansprüchen definiert ist, zu verlassen.

### Figurenbeschreibung

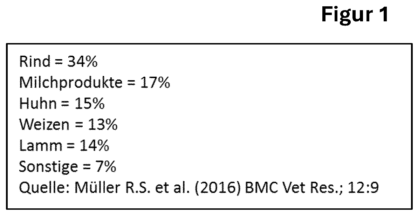

Figur 1 zeigt die häufigsten Allergene beim Hund
Quelle: Mueller, R. S., Olivry, T., & Prélaud, P. (2016). Critically appraised topic on adverse food reactions of companion animals (2): common food allergen sources in dogs and cats. BMC veterinary research, 12, 1-4.

## Patentansprüche

1. Vegane Tierfuttermittelzusammensetzung zur Verwendung zur Behandlung von Futtermittelallergien, entzündlichen Darmerkrankungen, Nierenerkrankungen, Lebererkrankungen, Bauchspeicheldrüsenerkrankungen, Hauterkrankungen und Übergewicht bei Tieren sowie zur purinarmen Ernährungvon Tieren, wobei die vegane Tierfuttermittelzusammensetzung enthält:
5 bis 60 Gew.-% bezogen auf das Trockengewicht der Zusammensetzung eines Einzelzellproteinprodukts aus der Biomasse einer Mikroorganismusspezies;
0,5 bis 65 Gew.-% bezogen auf das Trockengewicht der Zusammensetzung Kohlenhydrate nicht-tierischen Ursprungs;
0.5 bis 10 Gew.-% bezogen auf das Trockengewicht der Zusammensetzung Fette nicht-tierischen Ursprungs;
0,001 bis 20 Gew.-% bezogen auf das Trockengewicht der Zusammensetzung pflanzliche Faserstoffe.

2. Vegane Tierfuttermittelzusammensetzung nach Anspruch 1, wobei die Mikroorganismusspezies unter Bakterienspezies, Hefespezies, Pilzspezies und Protozoen ausgewählt sind.

3. Vegane Tierfuttermittelzusammensetzung nach Anspruch 2, wobei die Bakterienspezies aus der aus den folgenden Bakteriengattungen bestehenden Gruppe ausgewählt ist: Vibrio, Geobacillus, Bacillus, Cupriavidus, Lactococcus, Lactobacillus, Enterococcus, Streptococcus und Pediococcus.

4. Vegane Tierfuttermittelzusammensetzung nach Anspruch 3, wobei die Bakterienspezies aus der aus folgenden Spezies bestehenden Gruppe ausgewählt ist: Vibrio natriegens, Geobacillus LC300, Bacillus megaterium, Cupriavidus necator DSM 531, Cupriavidus necator DSM 541 und gentechnisch veränderte Mutanten davon.

5. Vegane Tierfuttermittelzusammensetzung nach Anspruch 2, wobei die Pilzspezies aus der aus den folgenden Pilzgattungen bestehenden Gruppe ausgewählt ist: Fusarium, Rhizopus, Aspergillus, Rasamsonia, Talaromyces, Penicillium, Acremonium, Humicola, Paecilomyces, Chaetomium, Rhizomucor, Rhizopus, Thermomyces, Myceliophthora, Thermoascus, Thielavia, Mucor, Stibella, Melanocarpus, Malbranchea, Dactylomyces, Canariomyces, Scytalidium, Mvriococcum, Corynascus und Coonemeria.

6. Vegane Tierfuttermittelzusammensetzung nach Anspruch 5, wobei die Pilzspezies aus der aus folgenden Spezies bestehenden Gruppe ausgewählt ist: Fusarium venenatum, Rhizopus oligosporus, Rhizopus oryzae, Aspergillus oryzae, Aspergillus sojae, Rhizomucor pusillus, Rhizomucor pusillus CBS 143028 und gentechnisch veränderte Mutanten davon.

7. Vegane Tierfuttermittelzusammensetzung nach einem der vorstehenden Ansprüche, wobei das Einzelzellproteinprodukt aus der aus einem Zelllysat, Proteinkonzentrat, Proteinisolat, Proteinextrakt, Proteinhydrolysat, freien Aminosäuren, Peptiden und Oligopeptiden und Kombinationen daraus bestehenden Gruppe ausgewählt ist.

8. Vegane Tierfuttermittelzusammensetzung nach einem der vorstehenden Ansprüche, wobei die Kohlenhydrate aus der aus Buchweizenkohlenhydrat, Dinkelkohlenhydrat, Amaranthkohlenhydrat, Quinoakohlenhydrat, Weizenkohlenhydrat, Hirsenkohlenhydrat, Süßkartoffelkohlenhydrat, Maniokkohlenhydrat, Roggenkohlenhydrat, Gerstenkohlenhydrat, Haferkohlenhydrat, Maiskohlenhydrat, Reiskohlenhydrat, Kartoffelkohlenhydrat und Kombinationen davon bestehenden Gruppe ausgewählt sind.

9. Vegane Tierfuttermittelzusammensetzung nach einem der vorstehenden Ansprüche, wobei die Fette aus der aus Sonnenblumenöl, Algenöl, Chiasamenöl, Sesamöl, Nachtkerzenöl, Kürbiskernöl, Traubenkernöl, Sanddornöl, Hagebuttenkernöl, Arganöl, Schwarzkümmelöl, Borretschöl, Aprikosenkernöl, Mandelöl, Erdnussöl, Leinöl, Leinsamenschrot, Leindotteröl, Olivenöl, Rapsöl, Maiskeimöl, Haselnussöl, Hanföl, Reiskeimöl, Sesamöl, Distelöl, Sojaöl, Palmöl, Kokosfett, Wallnussöl oder Kombinationen davon bestehenden Gruppe ausgewählt sind.

10. Vegane Tierfuttermittelzusammensetzung nach einem der vorstehenden Ansprüche, wobei die Faserstoffe unter Cellulose, Pektin, Hemicellulose und Lignin ausgewählt sind.

11. Vegane Tierfuttermittelzusammensetzung nach einem der vorstehenden Ansprüche, die zusätzlich essentielle Mineralstoffe, Spurenelemente und Vitamine enthält.

12. Vegane Tierfuttermittelzusammensetzung nach Anspruch 11, wobei die Mengenelemente aus der aus Natrium, Kalium, Calcium, Magnesium, Phosphor und Kombinationen daraus bestehenden Gruppe ausgewählt sind und/oder wobei die Spurenelemente aus der aus Eisen, Zink, Selen, Kupfer, Mangan, Jod, Fluor und Kombinationen daraus bestehenden Gruppe ausgewählt sind und/oder wobei die Vitamine aus der aus den fettlöslichen Vitaminen E, D, K, A, wasserlöslichen B-Vitaminen und Vitamin C bestehenden Gruppe ausgewählt sind.

13. Vegane Tierfuttermittelzusammensetzung nach einem der vorstehenden Ansprüche in Form von Trocken- oder Nassfutter.

14. Vegane Tierfuttermittelzusammensetzung nach einem der vorstehenden Ansprüche, wobei die Tiere Nutztiere und Heimtiere, insbesondere Hunde und Katzen, sind.

15. Vegane Tierfuttermittelzusammensetzung nach einem der vorstehenden Ansprüche 1 bis 13, wobei die Tiere Hunde sind.
